(19) Europäisches Patentamt / European Patent Office / Office européen des brevets

(11) **EP 4 327 738 A1**

(12) **EUROPEAN PATENT APPLICATION**

(43) Date of publication:
**28.02.2024 Bulletin 2024/09**

(21) Application number: **23191708.9**

(22) Date of filing: **16.08.2023**

(51) International Patent Classification (IPC):
**A61B 5/08** (2006.01) **A61B 5/1455** (2006.01)

(52) Cooperative Patent Classification (CPC):
**A61B 5/14551; A61B 5/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.08.2022 JP 2022135058**

(71) Applicants:
• **Nihon Kohden Corporation**
**Shinjuku-ku**
**Tokyo (JP)**

• **Hirasawa, Eiji**
**Fuchu-shi, Tokyo (JP)**

(72) Inventors:
• **HIRASAWA, Eiji**
**Fuchu-shi,, Tokyo (JP)**
• **ITO, Kazumasa**
**Tokorozawa-shi, Saitama (JP)**

(74) Representative: **Grünecker Patent- und Rechtsanwälte**
**PartG mbB**
**Leopoldstraße 4**
**80802 München (DE)**

(54) **RESPIRATION INFORMATION OBTAINING APPARATUS, RESPIRATION INFORMATION OBTAINING SYSTEM, PROCESSING APPARATUS, AND RESPIRATION INFORMATION OBTAINING METHOD**

(57) A respiration information obtaining apparatus includes: a light emitting unit configured to emit a first light including a first wavelength and a second light including a second wavelength different from the first wavelength; a light detecting unit that outputs a first signal and a second signal respectively corresponding to an intensity of the first light and an intensity of the second light after an interaction with a tissue of a subject, and a processor that calculates an index value based on a ratio between the first signal and the second signal, and obtains respiration information of the subject based on a change over time in the index value.

FIG. 1

EP 4 327 738 A1

**Description**

TECHNICAL FIELD

The invention relates to an apparatus and method for obtaining respiration information of a subject. The invention relates to a system including the apparatus and a sensor, and a processing apparatus mounted on the apparatus.

BACKGROUND ART

JP 2010-523248A discloses a sensor that obtains respiration information of a subject on the basis of the concentration of water vapor included in expired air of a subject.

It is required to enhance the convenience of a sensor that obtains respiration information of a subject.

SUMMARY

An aspect of the invention is a respiration information obtaining apparatus including:

a light emitting unit configured to emit a first light including a first wavelength and a second light including a second wavelength different from the first wavelength;
a light detecting unit that outputs a first signal and a second signal respectively corresponding to an intensity of the first light and an intensity of the second light after an interaction with a tissue of a subject, and
a processor configured to calculate an index value based on a ratio between the first signal and the second signal, and obtains respiration information of the subject based on a change over time in the index value.

An aspect of the invention is a respiration information obtaining system including:

a first sensor including a light emitting unit and a light detecting unit, and
a respiration information obtaining apparatus including a processor, in which
the light emitting unit emits a first light including a first wavelength and a second light including a second wavelength different from the first wavelength,
the light detecting unit outputs a first signal and a second signal respectively corresponding to an intensity of the first light and an intensity of the second light after interacting with a tissue of a subject, and
the processor configured to calculate an index value based on a ratio between the first signal and the second signal, and obtains first respiration information of the subject based on a change over time in the index value.

An aspect of the invention is a processing apparatus including:

an interface configured to receive a first signal and a second signal respectively corresponding to an intensity of a first light including a first wavelength and an intensity of a second light including a second wavelength different from the first wavelength after being emitted from a light emitting unit and interacting with a tissue of a subject; and
a processor configured to calculate an index value based on a ratio of the first signal and the second signal and obtain respiration information of the subject based on a change over time of the index value.

An aspect of the invention is a respiration information obtaining method including:

receiving, from a light detecting unit, a first signal and a second signal respectively corresponding to an intensity of a first light and an intensity of a second light after interacting with a tissue of a subject;
calculating an index value based on a ratio of the first signal and the second signal of the subject; and
obtaining respiration information of the subject based on a change over time of the index value.

According to the configuration according to each of the above-described aspect examples, the respiration information reflecting the gas exchange associated with the respiration of the subject can be obtained using the principle of the pulse photometry. Since it is not necessary to mount a sensor around the mouth or the nose of the subject as in the case of capnogram measurement, the burden on the subject and the medical worker can be reduced. Therefore, it is possible to enhance the convenience of the sensor that obtains the respiration information of the subject.

BRIEF DESCRIPTION OF DRAWINGS

FIG. 1 illustrates a configuration of a respiration information obtaining apparatus according to a first embodiment.
FIG. 2 illustrates a process executed by the processor of FIG. 1.
FIG. 3 illustrates a waveform of a signal processed by the processor of FIG. 1.
FIG. 4 illustrates a waveform of a signal processed by the processor of FIG. 1.
FIG. 5 illustrates a comparison between a waveform of a signal obtained through an actual process and a capnogram waveform.
FIG. 6 illustrates a part of a first detection signal and a part of a second detection signal processed by the processor of FIG. 1.

FIG. 7 illustrates a configuration of a respiration information obtaining system according to a second embodiment.

DESCRIPTION OF EMBODIMENTS

Hereinafter, an embodiment will be described in detail with reference to the accompanying drawings. In the drawings referred to in the following description, the scale is appropriately changed in order to make each element a recognizable size.

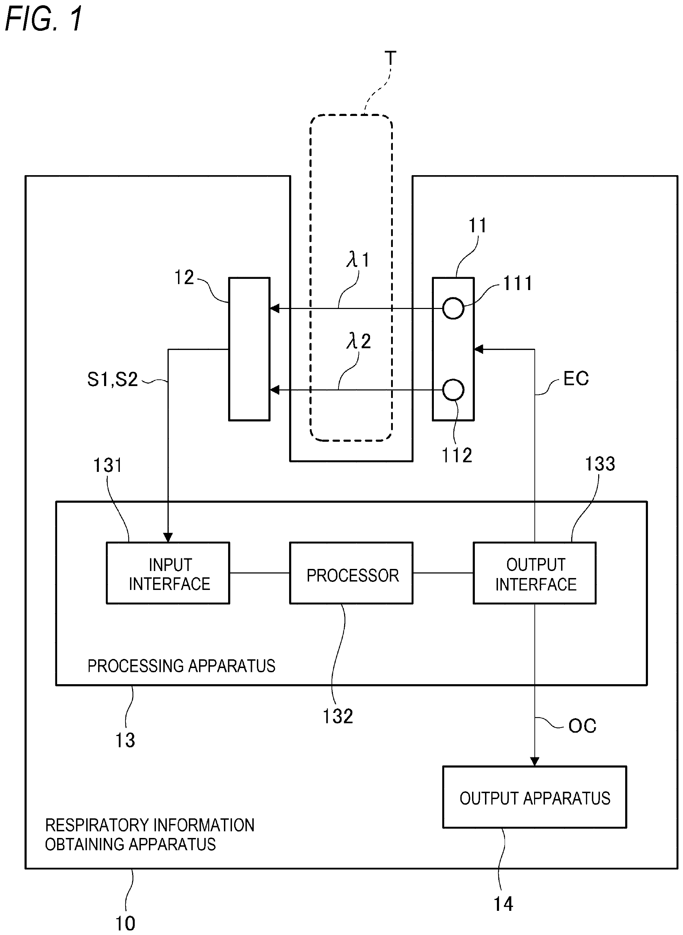

FIG. 1 illustrates a functional configuration of a respiration information obtaining apparatus 10 according to a first embodiment. The respiration information obtaining apparatus 10 includes a light emitting apparatus 11, a light detecting apparatus 12, a processing apparatus 13, and an output apparatus 14.

The light emitting apparatus 11 includes a first light emitter 111 and a second light emitter 112. The first light emitter 111 is configured to emit the first light including the first wavelength λ1 toward the tissue T of the subject. The second light emitter 112 is configured to emit a second light including a second wavelength λ2, for the tissue T. For example, the first wavelength λ1 may be included in the red wavelength band, and the second wavelength λ2 may be included in the infrared wavelength band.

Each of the first light emitter 111 and the second light emitter 112 may be a semiconductor light emitter. Examples of the semiconductor light emitter include light emitting diodes, laser diodes, and organic EL elements.

The light detecting apparatus 12 includes a light detecting element. The light detecting element is configured to output a first detection signal S1 and a second detection signal S2 having signal intensity respectively corresponding to the intensity of the first light and the intensity of the second light incident on the light-detecting surface. Each of the first detection signal S1 and the second detection signal S2 may be an analog signal or a digital signal. Examples of the light detecting element are a photodiode, a phototransistor, and a photoresistor.

The processing apparatus 13 includes an input interface 131. The input interface 131 is configured as a hardware interface that receives the first detection signal S1 and the second detection signal S2 output from the light detecting apparatus 12. When each of the first detection signal S1 and the second detection signal S2 is an analog signal, the input interface 131 includes an appropriate conversion circuit including an A / D converter.

The processing apparatus 13 includes one or more processor 132. The processor 132 is configured to execute processing for realizing various functions to be described later.

The above-described function of the processor 132 may be realized by a general-purpose microprocessor which operates in cooperation with one or more general-purpose memory. Examples of the general-purpose microprocessor may include CPU, MPU, and GPU. Examples of such a general-purpose memory are a RAM and a ROM. In this case, a computer program that executes the above processing can be stored in the ROM. The general-purpose microprocessor may designate at least some of the computer program stored in the ROM and develop it on the RAM, and execute the above process in cooperation with the RAM.

The processor 132 may be implemented by a dedicated integrated circuit capable of executing the computer program, such as a microcontroller, an ASIC, or an FPGA. In this case, the computer program is preinstalled in a storage element included in the dedicated integrated circuit. The processor 132 may be realized by a combination of a general-purpose microprocessor and a dedicated integrated circuit.

The processing apparatus 13 includes an output interface 133. The output interface 133 is configured as a hardware interface that outputs a control signal for causing the light emitting apparatus 11 and the output apparatus 14 to execute a predetermined operation. The control signal may be an analog signal or a digital signal. In a case where the control signal is an analog signal, the output interface 133 includes an appropriate conversion circuit including a D/A converter.

Specifically, the processor 132 outputs a light emission control signal EC for alternately emitting the first light and the second light to the light emitting apparatus 11 from the output interface 133. The first light emitter 111 and the second light emitter 112 alternately emit the first light and the second light at a timing specified by the light emission control signal EC.

The first light and the second light are alternately incident on the tissue T. The first light and the second light which interact with the tissue T are incident on the light-detecting surface of the light detecting element. The expression "light interacting with a tissue" used in the present specification means both light transmitted through the tissue and light reflected by the tissue.

Therefore, the light detecting apparatus 12 alternately outputs the first detection signal S1 and the second detection signal S2. The processor 132 of the processing apparatus 13 identifies which detection signal has been received by the input interface 131 based on the timing at which the light emission control of the first light emitter 111 and the second light emitter 112 is performed.

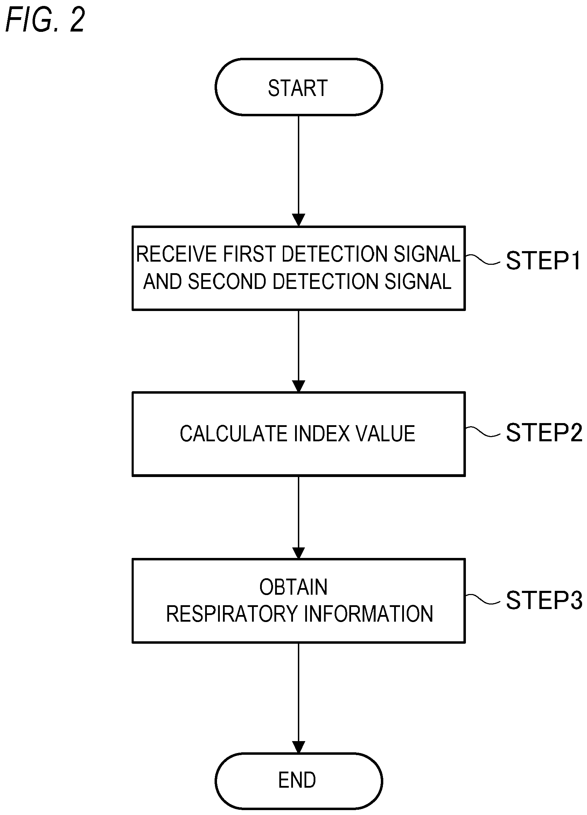

A method of obtaining respiration information executed by the processor 132 will be described with reference to FIG. 2.

First, as described above, the processor 132 alternately receives the first detection signal S1 and the second detection signal S2 through the input interface 131 (STEP 1).

Subsequently, the processor 132 calculates the index value $\Phi(t)$ at the time point t when the first detection signal S1 and the second detection signal S2 are obtained using the following expression (STEP 2).

$$\Phi(t)=G1(t)/G2(t)$$

$$G1(t)=\Delta S1(t)/S1(t)$$

$$G2(t)=\Delta S2(t)/S2(t)$$

$\Delta S1(t)$ represents the gradient of the first detection signal S1 at the time point t. $\Delta S1(t)$ can be calculated by, for example, the least square method using the value of the intensity of the first light obtained at a plurality of time points including S1(t).

Similarly, $\Delta S2(t)$ represents the gradient of the second detection signal S2 at the time point t. $\Delta S2(t)$ can be calculated by, for example, the least square method using the value of the intensity of the second light obtained at a plurality of time points including S2(t).

That is, the index value $\Phi(t)$ is calculated based on the ratio of the first detection signal S1 and the second detection signal S2 obtained at the time point t. In a case where the first light and the second light are alternately emitted as in the present example, strictly speaking, the time point at which the first detection signal S1 is obtained and the time point at which the second detection signal S2 is obtained are different. Therefore, a process of suppressing the influence of the time difference by advancing or delaying the phase of at least one of the first detection signal S1 and the second detection signal S2 may be performed as necessary.

In a case where the tissue T of the subject who receives the irradiation of the first light and the second light includes an artery, the first wavelength $\lambda1$ and the second wavelength $\lambda2$ can be selected as two wavelengths in which a significant difference is observed between the absorbance of the oxygenated hemoglobin contained in the arterial blood. The oxygenated hemoglobin is an example of a blood light absorber. In this case, the index value $\Phi$ has a correlation with the transcutaneous arterial oxygen saturation (SpO2) of the subject.

Subsequently, the processor 132 obtains the respiration information of the subject based on the change over time of the index value $\Phi$ (STEP 3).

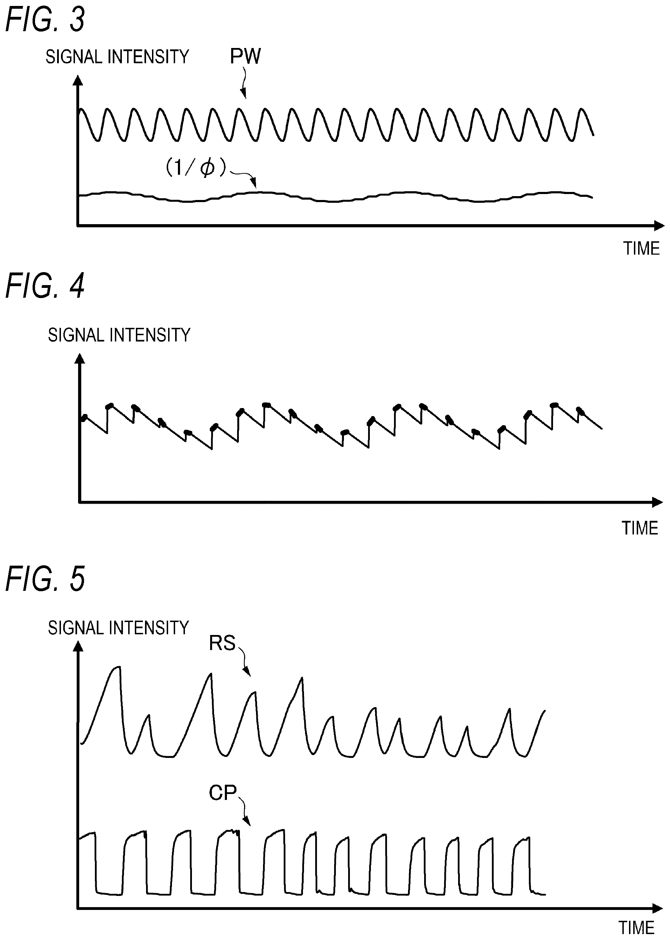

Since the index value $\Phi$ has a negative correlation with the SpO2, a value (1/$\Phi$) which is the reciprocal of the index value $\Phi$ is obtained. FIG. 3 illustrates a change over time of the pulse wave PW observed in the SpO2 measurement and the (1/$\Phi$) value. The vertical axis represents the magnitude of the relative signal intensity in each waveform, and does not compare the absolute values of the signal intensities of both waveforms. It should be noted that it is preferable that excluding a time section in which a change in the first detection signal S1 and the second detection signal S2 is small from a subject of the processing, or a noise removal accompanied by a relatively large variation in the index value $\Phi$

are appropriately performed.

As one implementation example, (1/$\Phi$) of the rising phase of each pulse wave is extracted. Since the (1/$\Phi$) value is obtained discretely for time, interpolation processing is performed. For example, by performing a process of subtracting a constant value from (1/$\Phi$) value obtained at a certain time point, a waveform in which the change over time of the (1/$\Phi$) value as illustrated in FIG. 4 is easily captured is obtained.

FIG. 5 illustrates the respiration waveform RS obtained by applying the same processing as the example of FIG. 4 to the pulse wave observed by the measurement of the SpO2 to the actual subject.

In FIG. 5, the respiration waveform (capnogram) CP of the subject obtained at the same time by using a well-known carbon dioxide gas concentration sensor (capnometer) is illustrated for comparison. The vertical axis represents the magnitude of the relative signal intensity in each waveform, and does not compare the absolute values of the signal intensities of both waveforms. Although the shape of the waveform is different, it can be seen that information such as the respiration rate is substantially reflected.

As illustrated in FIG. 1, the processor 132 outputs an output control signal OC for causing the output apparatus 14 to output the obtained respiration information from the output interface 133.

The output apparatus 14 is configured to output the obtained respiration information based on the output control signal OC. The information is configured to visually provide at least one of the respiratory waveform exemplified in FIG. 5 and the respiration rate per unit time calculated based on the respiratory waveform. The visual provision of the information may be provided as an image displayed on the display, or may be provided as a printed matter. In addition to or in place of this, a sound synchronized with the respiration rate and the respiration waveform may be audibly provided through a speaker.

According to the configuration of the present embodiment, the respiration information reflecting the gas exchange associated with the respiration of the subject can be obtained using the principle of the pulse photometry. Since it is not necessary to mount a sensor around the mouth or the nose of the subject as in the case of capnogram measurement, the burden on the subject and the medical worker can be reduced. Therefore, it is possible to enhance the convenience of the sensor that obtains the respiration information of the subject.

In particular, in a case where the first wavelength $\lambda1$ of the first light and the second wavelength $\lambda2$ of the second light irradiated to the tissue T as in the present embodiment are determined based on an absorption characteristic of the blood light absorber concentration in blood, respiration information can also be obtained with a configuration for measuring the blood light absorber concentration. Since the number of sensors mounted to obtain these pieces of information can be reduced, the burden on a patient or a medical worker can be reduced.

Examples of the blood light absorber include deoxygenated hemoglobin, carboxyhemoglobin, Met hemoglobin, and a dye in addition to the above-described oxygenated hemoglobin.

Note that measurement of the blood light absorber concentration in blood is not essential, and the first wavelength $\lambda 1$ and the second wavelength $\lambda 2$ may be appropriately selected as long as the change in gas exchange in the lungs in one respiration cycle is reflected in the index value $\Phi$ corresponding to the absorbance ratio of the tissue T.

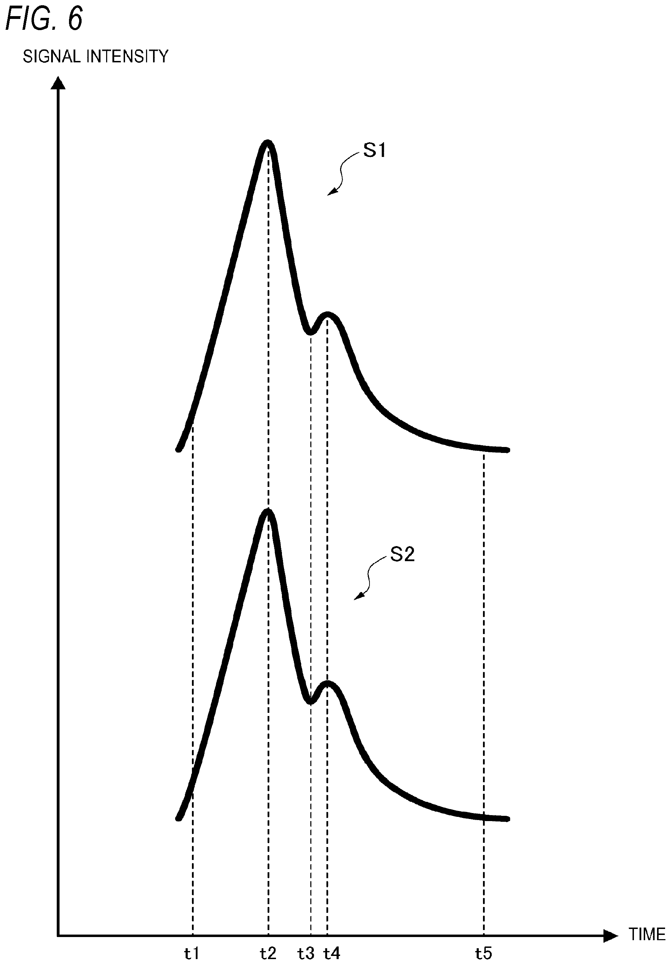

FIG. 6 illustrates a change over time in the first detection signal S 1 and a change over time in the second detection signal S2 corresponding to one pulsation of the subject. The vertical axis represents the magnitude of the relative signal intensity in each waveform, and does not compare the absolute values of the signal intensities of both waveforms. In calculating the blood light absorber concentration in blood such as the SpO2, it is common that the absorbance ratio obtained from the first detection signal S 1 and the second detection signal S2 is constant in one beat and is treated with a resolution per beat. On the other hand, in the present embodiment, as described above, the index value $\Phi$ is calculated based on the ratio of the gradient $\Delta S 1$ of the first detection signal S 1 and the gradient $\Delta S2$ of the second detection signal S2. According to such a configuration, the change over time of the index value $\Phi$ can be obtained with a resolution higher than that of the beat, so that it is possible to improve the accuracy of the respiration information obtained as a result of the subsequent signal processing.

From the viewpoint of calculating the index value $\Phi$ based on the significant ratio, it is preferable that the gradient $\Delta S1$ and the gradient $\Delta S2$ are obtained in the time section in which the positive and negative values match. In addition, it is preferable that a value is obtained for a time section in which both the absolute value of the gradient $\Delta S 1$ and the absolute value of the gradient $\Delta S2$ exceed the threshold. In the illustrated example, the section from the time point t3 to the time point t4 is excluded.

From the viewpoint of suppressing the influence of the venous return, it is more preferable that a value is obtained for a time section in which both the gradient $\Delta S1$ and the gradient $\Delta S2$ are positive. In the illustrated example, a section from a time point t4 to a time point t5 and the like are excluded. In addition, it is also assumed that the pulsation itself affects other components (veins, tissues, and the like), and it is also preferable to obtain values from the same phase in one beat from the viewpoint of suppressing or equalizing the influence of the other components. In the illustrated example, a section from t1 to t2 is employed.

In the present embodiment, the respiration information obtaining apparatus 10 includes a light emitting apparatus 11 and a light detecting apparatus 12. According to such a configuration, it is possible to enhance the portability of the apparatus capable of obtaining the respiration information.

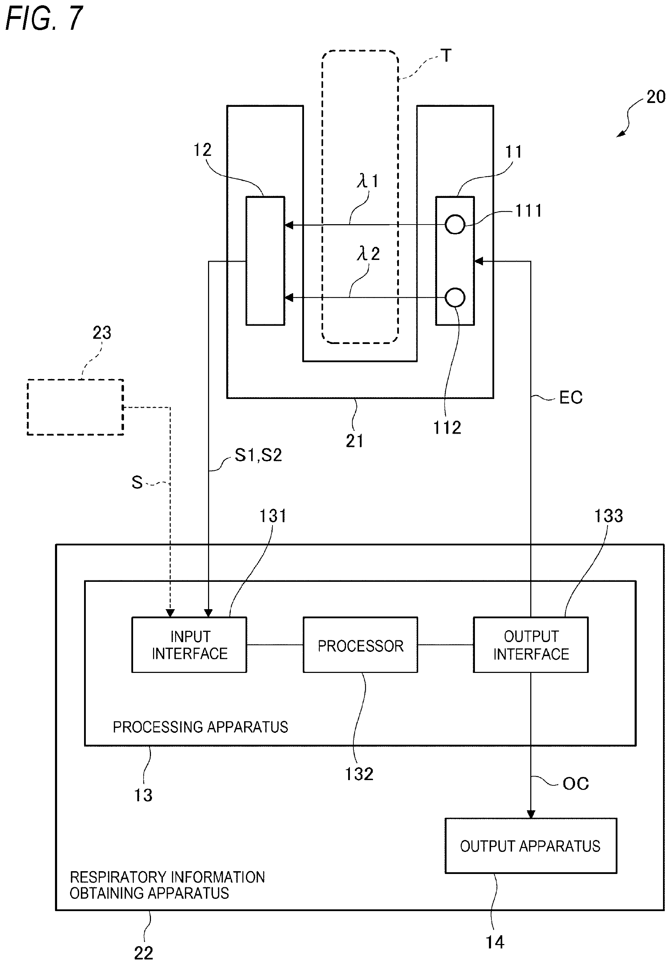

FIG. 7 illustrates a configuration of a respiration information obtaining system 20 according to a second embodiment. The respiration information obtaining system 20 includes a first sensor 21 and a respiration information obtaining apparatus 22. The first sensor 21 and the respiration information obtaining apparatus 22 are communicably connected to each other.

The first sensor 21 is configured to be attachable to the tissue T of the subject. The first sensor 21 includes the light emitting apparatus 11 and the light detecting apparatus 12 described with reference to FIG. 1. The respiration information obtaining apparatus 22 includes the processing apparatus 13 and the output apparatus 14 described with reference to FIG. 1. Since the operation of each apparatus is the same as that of the first embodiment, repetitive description will be omitted.

Even with such a configuration in which the light emitting apparatus 11 and the light detecting apparatus 12 are provided as an apparatus independent of the respiration information obtaining apparatus 22, the above-described effects can be obtained.

The respiration information obtaining system 20 may include a second sensor 23. The second sensor 23 and the respiration information obtaining apparatus 22 are communicably connected to each other. The second sensor 23 is configured to obtain respiration information of the subject by a method different from that of the first sensor 21. Examples of different methods include respiratory measurement by a respiratory motion such as an impedance method.

In this case, the processor 132 of the respiration information obtaining apparatus 22 may be configured to obtain first respiration information indicating whether the subject is in the apnea state based on the respiration waveform obtained using the index value $\Phi$.

On the other hand, the detection signal S transmitted from the second sensor 23 may include second respiration information indicating whether the subject obtained by a method different from that of the first sensor 21 is in an apnea state. The processor 132 obtains the second respiration information when the detection signal S is received by the input interface 131.

The second sensor 23 may transmit only a signal corresponding to the detected respiratory state of the subject, and the processor 132 may determine whether the subject is in an apnea state based on the signal.

The processor 132 may be configured to estimate the type of apnea syndrome that the subject develops based on the combination of the first respiration information and the second respiration information. Examples of the type of apnea syndrome include a central property, an occlusion property, and a composite property.

As an example, when both the first respiration information and the second respiration information indicate that the subject is in the apnea state, the processor 132 estimates that the subject has developed the central apnea syndrome.

As another example, when the first respiration information indicates that the subject is in the apnea state and the second respiration information indicates that the subject is not in the apnea state, the processor 132 estimates that the subject has developed the obstructive apnea syndrome.

The processor 132 outputs an output control signal OC for causing the output apparatus 14 to output the estimation result from the output interface 133. The output of the estimation result by the output apparatus 14 may be performed through at least one of a visual output, an auditory output, and a haptic output.

Each of the configurations described above is merely an example for easy understanding of the invention. The respective configurations may be appropriately changed or combined with other configurations without departing from the gist of the invention.

In each of the above-described configuration examples, the processing apparatus 13 is mounted on the respiration information obtaining apparatus. However, the processing apparatus 13 may be installed in an apparatus capable of data communication with the respiration information obtaining apparatus. In this case, the respiration information obtaining apparatus includes a communication apparatus for performing data communication with the processing apparatus 13. The first detection signal S1 and the second detection signal S2 output from the light detecting apparatus 12 are transmitted to the processing apparatus 13 by the communication apparatus. The processing apparatus 13 executes the various processes described above and transmits the output control signal OC to the respiration information obtaining apparatus. The respiration information obtaining apparatus that has received the output control signal OC outputs the obtained respiration information from the output apparatus 14.

In each of the embodiments described above, the light emitting apparatus 11 that emits the first light including the first wavelength λ1 and the second light including the second wavelength λ2 is used. However, the light emitting apparatus 11 capable of emitting light including three or more different wavelengths may be used. In this case, the two wavelengths used for the calculation of the index value Φ can be arbitrarily selected from three or more wavelengths.

The configurations listed below also constitute a part of the invention.

(1): A respiration information obtaining apparatus including:

a light emitting unit configured to emit a first light including a first wavelength and a second light including a second wavelength different from the first wavelength;
a light detecting unit that outputs a first signal and a second signal respectively corresponding to an intensity of the first light and an intensity of the second light after an interaction with a tissue of a subject, and
a processor configured to calculate an index value based on a ratio between the first signal and the second signal, and obtains respiration information of the subject based on a change over time in the index value.

(2): The respiration information obtaining apparatus according to (1), in which the first wavelength and the second wavelength are determined based on an absorption characteristic of a blood light absorber of the subject.

(3): The respiration information obtaining apparatus according to (1) or (2), in which the index value is calculated based on a ratio of a first gradient, which is a gradient of a change over time of the first signal, and a second gradient, which is a gradient of a change over time of the second signal.

(4): The respiration information obtaining apparatus according to (3), in which the index value is calculated by using a section in which a positive or negative sign of the first gradient and a positive or negative sign of the second gradient coincide with each other, an absolute value of the first gradient exceeds a first threshold, and an absolute value of the second gradient exceeds a second threshold.

(5): The respiration information obtaining apparatus according to (4), in which the index value is calculated using a section in which both the first gradient and the second gradient are positive.

(6): The respiration information obtaining apparatus according to (4), in which the index value is calculated using the first gradient and the second gradient obtained from a section corresponding to a same phase in one beat.

(7): The respiration information obtaining apparatus according to any one of (1) to (6), in which the respiration information includes at least one of a respiration rate and a respiration curve.

(8): A respiration information obtaining system including:

a first sensor including a light emitting unit and a light detecting unit, and
a respiration information obtaining apparatus including a processor, in which
the light emitting unit emits a first light including a first wavelength and a second light including a second wavelength different from the first wavelength,
the light detecting unit outputs a first signal and a second signal respectively corresponding to the intensity of the first light and the intensity of the second light after interacting with a tissue of a subject, and
the processor configured to calculate an index value based on a ratio between the first signal and the second signal, and obtains first respiration information of the subject based on a change over time in the index value.

(9): The respiration information obtaining system according to (8), further including:

a second sensor configured to obtain second respiration information of the subject by a method different from that of the first sensor, in which the processing apparatus is configured to estimate a type of an apnea syndrome developed by the subject based on a combination of the first respiration information and the second respiration information.

(10): A processing apparatus including:

an interface configured to receive a first signal and a second signal respectively corresponding to an intensity of a first light including a first wavelength and an intensity of a second light including a second wavelength different from the first wavelength after being emitted from a light emitting unit and interacting with a tissue of a subject; and

a processor configured to calculate an index value based on a ratio of the first signal and the second signal and obtain respiration information of the subject based on a change over time of the index value.

(11): A respiration information obtaining method including:

receiving, from a light detecting unit, a first signal and a second signal respectively corresponding to an intensity of a first light and an intensity of a second light after interacting with a tissue of a subject;
calculating an index value based on a ratio of the first signal and the second signal of the subject; and
obtaining respiration information of the subject based on a change over time of the index value.

REFERENCE SIGNS LIST

10: respiration information obtaining apparatus
11: light emitting apparatus
12: light detecting apparatus
13: processing apparatus
131: input interface
132: processor
20: respiration information obtaining system
21: first sensor

22: respiration information obtaining apparatus
23: second sensor
S1: first detection signal
S2: second detection signal
T: tissue
$\lambda 1$: first wavelength
$\lambda 2$: second wavelength
$\Delta S1$, $\Delta S2$: gradient
$\Phi$: index value

Claims

1. A respiration information obtaining apparatus comprising:

a light emitting unit configured to emit a first light including a first wavelength and a second light including a second wavelength different from the first wavelength;
a light detecting unit that outputs a first signal and a second signal respectively corresponding to an intensity of the first light and an intensity of the second light after an interaction with a tissue of a subject, and
a processor configured to calculate an index value based on a ratio between the first signal and the second signal, and obtains respiration information of the subject based on a change over time in the index value.

2. The respiration information obtaining apparatus according to claim 1, wherein
the first wavelength and the second wavelength are determined based on an absorption characteristic of a blood light absorber of the subject.

3. The respiration information obtaining apparatus according to claim 1, wherein
the index value is calculated based on a ratio of a first gradient, which is a gradient of a change over time of the first signal, and a second gradient, which is a gradient of a change over time of the second signal.

4. The respiration information obtaining apparatus according to claim 3, wherein
the index value is calculated by using a section in which a positive or negative sign of the first gradient and a positive or negative sign of the second gradient coincide with each other, an absolute value of the first gradient exceeds a first threshold, and an absolute value of the second gradient exceeds a second threshold.

5. The respiration information obtaining apparatus according to claim 4, wherein
the index value is calculated using a section in which both the first gradient and the second gradient are positive.

6. The respiration information obtaining apparatus according to claim 4, wherein
the index value is calculated using the first gradient and the second gradient obtained from a section corresponding to a same phase in one beat.

7. The respiration information obtaining apparatus according to claim 1, wherein
the respiration information includes at least one of a respiration rate and a respiration curve.

8. A respiration information obtaining system comprising:

a first sensor including a light emitting unit and a light detecting unit, and
a respiration information obtaining apparatus including a processor, wherein
the light emitting unit emits a first light including a first wavelength and a second light including a second wavelength different from the first wavelength,
the light detecting unit outputs a first signal and a second signal respectively corresponding to an intensity of the first light and an intensity of the second light after interacting with a tissue of a subject, and
the processor configured to calculate an index value based on a ratio between the first signal and the second signal, and obtains first respiration information of the subject based on a change over time in the index value.

9. The respiration information obtaining system according to claim 8, further comprising:

a second sensor configured to obtain second respiration information of the subject by a method different from that of the first sensor, wherein
the processing apparatus is configured to estimate a type of an apnea syndrome developed by the subject based on a combination of the first respiration information and the second respiration information.

10. A processing apparatus comprising:

an interface configured to receive a first signal and a second signal respectively corresponding to an intensity of a first light including a first wavelength and an intensity of a second light including a second wavelength different from the first wavelength after being emitted from a light emitting unit and interacting with a tissue of a subject; and

a processor configured to calculate an index value based on a ratio of the first signal and the second signal and obtain respiration information of the subject based on a change over time of the index value.

11. A respiration information obtaining method comprising:

receiving, from a light detecting unit, a first signal and a second signal respectively corresponding to an intensity of a first light and an intensity of a second light after interacting with a tissue of a subject;
calculating an index value based on a ratio of the first signal and the second signal of the subject; and
obtaining respiration information of the subject based on a change over time of the index value.

*FIG. 1*

T

λ1

12

λ2

11

111

S1,S2

EC

112

131

133

| INPUT INTERFACE | PROCESSOR | OUTPUT INTERFACE |

PROCESSING APPARATUS

13

132

OC

OUTPUT APPARATUS

RESPIRATORY INFORMATION
OBTAINING APPARATUS

14

10

*FIG. 2*

```
          ┌─────────────┐
          │    START    │
          └─────────────┘
                 │
                 ▼
  ┌──────────────────────────────┐
  │  RECEIVE FIRST DETECTION SIGNAL│──── STEP1
  │  AND SECOND DETECTION SIGNAL   │
  └──────────────────────────────┘
                 │
                 ▼
  ┌──────────────────────────────┐
  │     CALCULATE INDEX VALUE      │──── STEP2
  └──────────────────────────────┘
                 │
                 ▼
  ┌──────────────────────────────┐
  │           OBTAIN               │──── STEP3
  │   RESPIRATORY INFORMATION      │
  └──────────────────────────────┘
                 │
                 ▼
          ┌─────────────┐
          │     END     │
          └─────────────┘
```

## FIG. 3

SIGNAL INTENSITY      PW

(1 / $\phi$)

TIME

## FIG. 4

SIGNAL INTENSITY

TIME

## FIG. 5

SIGNAL INTENSITY      RS

CP

TIME

# FIG. 6

# FIG. 7

Europäisches
Patentamt

European
Patent Office

Office européen
des brevets

# EUROPEAN SEARCH REPORT

**Application Number**

EP 23 19 1708

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/219882 A1 (HUIKU MATTI VELI TAPANI [FI] ET AL) 22 July 2021 (2021-07-22) | 1-8,10, 11 | INV. A61B5/08 A61B5/1455 |
| Y | * claim 9 * <br> * paragraph [0107] * <br> * paragraph [0036] * <br> * paragraph [0063] * <br> * paragraph [0035] * <br> * paragraph [0062] - paragraph [0065] * <br> * paragraph [0087] * <br> * claim 14 * | 9 | |
| Y | KR 2011 0088138 A (NAT UNIV CHUNGBUK IND ACAD [KR]) 3 August 2011 (2011-08-03) <br> * paragraph [0039] * | 9 | |
| A | US 2014/323874 A1 (ADDISON PAUL S [GB] ET AL) 30 October 2014 (2014-10-30) <br> * paragraph [0092] * | 3 | |
| A | WO 2022/046939 A1 (UNIV SOUTHERN CALIFORNIA [US]) 3 March 2022 (2022-03-03) <br> * paragraph [0005]; claim 1 * | 9 | TECHNICAL FIELDS SEARCHED (IPC) <br><br> A61B |

The present search report has been drawn up for all claims

| Place of search | Date of completion of the search | Examiner |
|---|---|---|
| The Hague | 1 November 2023 | Lai, Marco |

EPO FORM 1503 03.82 (P04C01)

## ANNEX TO THE EUROPEAN SEARCH REPORT
## ON EUROPEAN PATENT APPLICATION NO.

EP 23 19 1708

This annex lists the patent family members relating to the patent documents cited in the above-mentioned European search report.
The members are as contained in the European Patent Office EDP file on
The European Patent Office is in no way liable for these particulars which are merely given for the purpose of information.

01-11-2023

| Patent document cited in search report | | Publication date | Patent family member(s) | | Publication date |
|---|---|---|---|---|---|
| US 2021219882 | A1 | 22-07-2021 | NONE | | |
| KR 20110088138 | A | 03-08-2011 | NONE | | |
| US 2014323874 | A1 | 30-10-2014 | US | 2014323824 A1 | 30-10-2014 |
| | | | US | 2014323874 A1 | 30-10-2014 |
| | | | WO | 2014176576 A1 | 30-10-2014 |
| WO 2022046939 | A1 | 03-03-2022 | NONE | | |

For more details about this annex : see Official Journal of the European Patent Office, No. 12/82

EPO FORM P0459

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010523248 A